# EUROPEAN PATENT SPECIFICATION

(11) **EP 2 327 426 B1**
(45) Date of publication and mention of the grant of the patent: **29.04.2015**
(21) Application number: 10251909.7
(22) Date of filing: 09.11.2010
(51) Int. Cl.: A61L 15/28, A61L 15/42, A61L 15/60

(54) **Hemostatic tapes and dispensers thereof**
Hämostatische Bänder und Spender dafür
Bande hémostatique et distributeur associé

(30) Priority: 10.11.2009 US 259836 P; 19.10.2010 US 907340
(43) Date of publication of application: 01.06.2011
(73) Proprietor: Covidien LP, Mansfield, MA 02048 (US)
(72) Inventor: Miller, Jeffrey, East Haven, CT 06512 (US)
(74) Representative: Soames, Candida Jane

(56) References cited:
- EP-A2- 2 179 753
- WO-A2-2010/043980
- US-A- 5 201 745
- US-A1- 2002 106 409
- US-A1- 2003 031 697

## Description

### BACKGROUND

### Technical Field

The present disclosure relates to hemostatic tapes, and more particularly, to hemostatic tapes which include a porous substrate removably attached to a release sheet, wherein the porous substrate has a first hydrogel precursor and a second hydrogel precursor applied thereto.

### Background of Related Art

Use of anticoagulant medications in the prevention of heart attack and stroke may be commonplace. The mode of action of these drugs involves thinning of the blood and inhibition of clotting. While useful with respect to heart attack or stroke prevention, these medications increase the risk of severe bleeding in patients.

For patients taking anticoagulants, everyday activities such as shaving can lead to nicks and cuts that are difficult to stop from bleeding. Scrapes or minor cuts can lead to excessive bleeding. Even minor injuries must be watched for bleeding.

In order to stop the bleeding of a minor cut, patients taking anticoagulants are often instructed to apply constant pressure over the cut until the bleeding stops. If the bleeding does not stop within 20 minutes, the patient may be further instructed to continue to apply pressure and go to the nearest emergency room.

Therefore, devices which seal the bleeding from minor cuts in patients on anticoagulant therapy would be useful.

### SUMMARY

The present disclosure describes hemostatic tapes which include a porous substrate; a first hydrogel precursor; a second hydrogel precursor; and a release sheet. The first and second hydrogel precursors may be applied to the porous substrate. In embodiments, at least one of the first and second hydrogel precursors may be applied to the porous substrate in particle form. The first hydrogel precursor is spatially separated from the second hydrogel precursor to prevent the first and second hydrogel precursors from reacting with each other until the tape may be positioned on the wound and exposed to the physiological fluids of the patient. Exposure of the tape to moisture, including physiological fluids, may cause the first hydrogel precursor to migrate through the porous substrate to react with the second hydrogel precursor. The release sheet may be removably attached to the porous substrate to prevent the tape from sticking prematurely. For example when being handled prior to being applied to a wound or while wound on a spool to form a tape roll. In embodiments, the tapes described herein display not only hemostatic properties but also display adhesive properties.

The present disclosure further describes packages for storing and dispensing of the hemostatic tapes. In embodiments, the package may be a tape dispenser which includes a housing and a roll of a hemostatic tape. The tape includes a porous substrate, a first hydrogel precursor and a second hydrogel precursor applied to the porous substrate, and a release sheet. The dispenser also includes a first support positioned on the housing for supporting the roll of hemostatic tape and a second support positioned on the housing configured to receive a length of the hemostatic tape which extends from the roll on the support. A cutting member is operatively coupled with the housing. The cutting member is configured to separate at least a portion of the length of tape from the roll.

In another embodiment, a hemostatic tape dispenser is disclosed which includes a housing having a base and at least one side wall. The housing is configured to receive a plurality of hemostatic tapes in a stacked configuration. Each tape includes a porous substrate, a first hydrogel precursor, and a second hydrogel precursor applied to the porous substrate, and a release sheet. A backing sheet may also be added to the tape. A biasing member having a proximal and distal end is positioned within the housing. The distal end of the biasing member is positioned near the base of the housing and the proximal end of the biasing member is positioned beneath a first support member. The first support member supports the stack of hemostatic tapes. A retaining member is positioned on a proximal end of the at least one side wall to retain the hemostatic tapes within the housing. An opening is positioned distal to the retaining member to allow for the lateral removal of the tape from the housing.

In another embodiment, a hemostatic tape dispenser is described which includes a housing configured to receive a roll of hemostatic tape, wherein the roll includes a backing sheet and a porous substrate. The porous substrate contains a first hydrogel precursor, a second hydrogel precursor and a release sheet. The dispenser has a first support member positioned on the housing for supporting the roll of hemostatic tape. A second support member is positioned on the housing and is configured to receive a length of the hemostatic tape which extends from the first support member. The dispenser also includes a slot positioned near the second support member, wherein the slot is configured for the passage of the hemostatic tape to allow for the removal of the porous substrate from the backing sheet. A third support member is positioned on the housing and the third support member is configured to receive a length of the backing sheet which extends from the second support member. The dispenser may also include a wheel member rotatably positioned on the housing and connected to the third support member, the wheel member rotatable to draw the length of tape from the first and second support members.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiments given below, serve to explain the principles of the disclosure.

Figures 1A-E schematically show the application of first and second hydrogel precursors to a porous substrate as described in at least one of the embodiments in the present disclosure;

Figure 2 shows a side view of a porous substrate in another embodiment of the present disclosure;

Figure 3 shows a side view of a porous substrate in yet another embodiment of the present disclosure;

Figures 4A-C schematically show the application of a first hydrogel precursor to a porous substrate as described in at least one of the embodiments in the present disclosure;

Figures 5A-D schematically show the application of particles including a second hydrogel precursor to a porous substrate already having a first hydrogel precursor applied thereto as described in at least one of the embodiments in the present disclosure;

Figures 6A-C schematically show the application of a film containing a second hydrogel precursor to a porous substrate already having a first hydrogel precursor applied thereto as described in at least one of the embodiments in the present disclosure;

Figures 7A-B schematically show the simultaneous formation of a foam containing a first hydrogel precursor and a foam porous substrate; and

Figures 8A-C schematically show the application of particles including a second hydrogel precursor to a porous substrate already having a first hydrogel precursor applied thereto as described in at least one of the embodiments in the present disclosure;

Figures 9A-C schematically show the application of a film containing a second hydrogel precursor to a porous substrate already having a first hydrogel precursor applied thereto as described in at least one of the embodiments in the present disclosure;

Figures 10 shows a side view of a tape in a roll configuration, wherein the tape has a first and second hydrogel precursors applied thereto and a release sheet and backing sheet attached to the porous substrate, as described in at least one of the embodiments of the present disclosure;

Figure 11 shows a side view of a dispenser designed and configured to receive one of the tapes described herein according to yet another embodiment of the present disclosure;

Figures 12A -B show side views of a dispenser designed and configured to receive one of the tapes described herein according to still another embodiment in the present disclosure;

Figures 13A-C show a dispenser designed and configured to receive a stack of individual tapes described herein according to another embodiment of the present disclosure; and

Figures 14A-C show a dispenser designed and configured to dispense individual tapes described herein according to yet another embodiment of the present disclosure.

### DETAILED DESCRIPTION

Hemostatic tapes in accordance with the present disclosure include a porous substrate attached to a release sheet, wherein the porous substrate includes a first hydrogel precursor and a second hydrogel precursor applied thereto. During use, the first and second hydrogel precursors react upon exposure to physiological fluids to provide hemostasis and adhere the tape on injured tissue. In some embodiments, the tape may be wrapped around tissue, such as organ. In some embodiments, the first and second hydrogel precursors may be distinguishable from one another by the addition of contrast dyes, surface texturing, coloring or other visual cues. The first and second hydrogel precursors are positioned on different portions of the porous substrate. Upon contact with an open wound, the porous substrate may soak up physiological fluids, such as blood, and the first hydrogel precursor may be dissolved by the fluid. As the fluid wicks into and migrates across the porous tape, it will carry the dissolved first hydrogel precursor along through the tape. Eventually, the fluid will migrate through the tape sufficiently to reach a second portion of the porous substrate to which the second hydrogel precursor may be applied, thereby dissolving the second hydrogel precursor. The first and second hydrogel precursors may then react to form a biocompatible cross-linked material, thereby adhering the tape to the tissue and assisting with hemostasis.

The porous substrate of the tape has openings or pores over at least a portion of a surface thereof. As described in more detail below, suitable materials for forming the porous substrate include, but are not limited to fibrous structures (e.g., knitted structures, woven structures, non-woven structures, etc.) and/or foams (e.g., open or closed cell foams). In some embodiments, the pores may be in sufficient number and size so as to interconnect across the entire thickness of the porous substrate. Woven fabrics, knitted fabrics and open cell foams are illustrative examples of structures in which the pores can be in sufficient number and size so as to interconnect across the entire thickness of the porous substrate. In other embodiments, the pores do not interconnect across the entire thickness of the porous substrate. Closed cell foams or fused non-woven materials are illustrative examples of structures in which the pores may not interconnect across the entire thickness of the porous substrate. The pores of the foam porous substrate may span across the entire thickness of porous substrate. In yet other embodiments, the pores may not extend across the entire thickness of the porous substrate, but rather may be present at a portion of the thickness thereof. In some embodiments, the openings or pores may be located on a portion of the surface of the porous substrate, with other portions of the porous substrate having a non-porous texture. Those skilled in the art reading the present disclosure will envision other pore distribution patterns and configurations for the porous substrate.

Where the porous substrate is fibrous, the fibers may be filaments or threads suitable for knitting or weaving or may be staple fibers, such as those frequently used for preparing non-woven materials. The fibers may be made from any biocompatible material. Thus, the fibers may be formed from a natural material or a synthetic material. The material from which the fibers are formed may be bioabsorbable or non-bioabsorbable. It should of course be understood that any combination of natural, synthetic, bioabsorbable and non-bioabsorbable materials may be used to form the fibers. Some non-limiting examples of materials from which the fibers or foams may be made include, but are not limited to poly(lactic acid), poly(glycolic acid), poly(trimethylene carbonate), poly(dioxanone), poly(hydroxybutyrate), poly(phosphazine), polyesters, polyethylene terephthalate, ultra-high molecular weight polyethylene, polyethylene glycols, polyethylene oxides, polyacrylamides, polyhydroxyethylmethylacrylate, polyvinylpyrrolidone, polyvinyl alcohols, polyacrylic acid, polyacetate, polycaprolactone, polypropylene, aliphatic polyesters, glycerols, poly(amino acids), copoly(ether-esters), polyalkylene oxalates, polysaccharides, polyamides, poly(iminocarbonates), polyalkylene oxalates, polyoxaesters, polyorthoesters, polyphosphazenes, biopolymers, polymer drugs and copolymers, block copolymers, homopolymers, blends and combinations thereof.

Where the porous substrate is fibrous, the porous substrate may be formed using any method suitable to forming fibrous structures, including but not limited to knitting, weaving, non-woven techniques, wet-spinning, electro-spinning, extrusion, coextrusion, and the like. Suitable techniques for making fibrous structures are within the purview of those skilled in the art.

In embodiments, the porous substrate may be made from fibers of oxidized cellulose. Such materials are known and include oxidized cellulose hemostat materials commercially available under the trade name SURGICEL^{®}. Methods for preparing oxidized cellulose hemostat materials are known to those skilled in the art and are disclosed, for example in U.S. Patent Nos. 3,364,200; 4,626,253; 5,484,913; and 6,500,777.

Where the porous substrate is a foam, the porous substrate may be formed using any method suitable to forming a foam or sponge including, but not limited to the lyophilization or freeze-drying of a composition. The foam may be cross-linked or non-cross-linked, and may include covalent or ionic bonds. Suitable techniques for making foams are within the purview of those skilled in the art.

The porous substrate may be at least 0.1 cm thick, in some embodiments from about 0.2 to about 1.5 cm thick. The porous substrate may be at least 0.1 cm in width, in some embodiments from about 0.2 to about 10 cm in width. In other embodiments, the tapes described herein may be manufactured into long narrow strips which may be rolled onto a spool. For example, the tape may be manufactured into a long strip capable of being rolled and measuring about 0.5 cm wide and at least about 10 cm in length. It is envisioned that the length and width of the tape may vary according to the size of a wound intended to be covered by the tape.

The size of the pores in the porous substrate may be from about 2 µm to about 300 µm, in some embodiments from about 50 µm to about 150 µm. It is envisioned that the pores of the substrate may be arranged in any manner in the substrate. For example, the pores may be configured in a random or uniform manner. In some embodiments, the pores may be formed with the use of copper alginate to create a honeycomb shaped porous substrate. In still other embodiments, the pores may be configured to create a gradient in the porous substrate. The gradient may further enhance the porous substrates ability to absorb the physiologic fluid and direct the migration of the physiological fluid carrying the first hydrogel precursor towards the second hydrogel precursor.

In some embodiments, the tape may be made from non-denatured collagen or collagen which has at least partially lost its helical structure through heating or any other method, consisting mainly of non-hydrolyzed α chains, of molecular weight close to 100 kDa. The term "non-denatured collagen" means collagen which has not lost its helical structure. The collagen used for the tape may be native collagen or atelocollagen, notably as obtained through pepsin digestion and/or after moderate heating as defined previously. The collagen may have been previously chemically modified by oxidation, methylation, ethylation, succinylation or any other known process. The collagen may also be cross-linked with any suitable crosslinker, such as genipin, isocyanates, and aldehydes. The collagen may also be combined with any suitable degradable and/or nondegradable material.

In embodiments, the tape may be obtained by freeze-drying an aqueous acid solution of collagen at a concentration of 2 to 50 g/l and an initial temperature of 4 to 25° C. The concentration of collagen in the solution may be from about 1 g/l to about 30 g/l, in embodiments about 10 g/l. This solution may be advantageously neutralized to a pH of around 6 to 8.

The tape may also be obtained by freeze-drying a fluid foam prepared from a solution of collagen or heated collagen, emulsified in the presence of a volume of air in variable respective quantities (volume of air:water varying from about 1 to about 10).

The porous substrate has a first hydrogel precursor applied thereto and a second hydrogel precursor applied thereto. The terms "first hydrogel precursor" and "second hydrogel precursor" each means a polymer, functional polymer, macromolecule, or small molecule that can take part in a reaction to form a network of crosslinked molecules, e.g. a hydrogel.

In embodiments, at least one of the first or second hydrogel precursors may be a small molecule of about 1000 Da or less, and may have a solubility of at least 1 g/100 mL in an aqueous solution. A crosslinked molecule may be crosslinked via an ionic or covalent bond, a physical force, or other attraction.

In embodiments, at least one of the first or second hydrogel precursors may be a macromolecule, and may be referred to as a "functional polymer". The macromolecule, when reacted in combination with a crosslinker, may be at least five to fifty times greater in molecular weight than the small molecule crosslinker and can be less than about 60,000 Da. In some embodiments, a macromolecule that may be seven to less than about 60,000 Da. In some embodiments, a macromolecule that may be seven to thirty times greater in molecular weight than the crosslinker may be used and, in some embodiments a macromolecule that may be about ten to twenty times difference in weight may be used. Further, a macromolecular molecular weight of 5,000 to 50,000 may be useful. The term polymer, as used herein, means a molecule formed of at least three repeating groups.

Each of the first and second hydrogel precursors may be multifunctional, meaning that it comprises two or more electrophilic or nucleophilic functional groups, such that, for example, a nucleophilic functional group on the first hydrogel precursor may react with an electrophilic functional group on the second hydrogel precursor to form a covalent bond. At least one of the first or second hydrogel precursors includes more than two functional groups, so that, as a result of electrophilic-nucleophilic reactions, the precursors combine to form crosslinked polymeric products. Such reactions are referred to as "crosslinking reactions".

In embodiments, each of the first and second hydrogel precursors includes only one category of functional groups, either only nucleophilic groups or only electrophilic functional groups, so long as both nucleophilic and electrophilic precursors are used in the crosslinking reaction. Thus, for example, if the first hydrogel presursor has nucleophilic functional groups such as amines, the second hydrogel precursor may have electrophilic functional groups such as N-hydroxysuccinimides. On the other hand, if first hydrogel precursor has electrophilic functional groups such as sulfosuccinimides, then the second hydrogel precursor may have nucleophilic functional groups such as amines or thiols. Thus, functional polymers such as proteins, poly(allyl amine), styrene sulfonic acid, or amine-terminated di- or multifunctional poly(ethylene glycol) ("PEG") can be used.

The first and second hydrogel precursors may have biologically inert and water soluble cores. When the core is a polymeric region that is water soluble, polymers that may be used include: polyether, for example, polyalkylene oxides such as polyethylene glycol("PEG"), polyethylene oxide ("PEO"), polyethylene oxide-co-polypropylene oxide ("PPO"), co-polyethylene oxide block or random copolymers, and polyvinyl alcohol ("PVA"); poly(vinyl pyrrolidinone) ("PVP"); poly(amino acids); poly (saccharides), such as dextran, chitosan, alginates, carboxymethylcellulose, oxidized cellulose, hydroxyethylcellulose, hydroxymethylcellulose, hyaluronic acid, and proteins such as albumin, collagen, casein, and gelatin. The polyethers and more particularly poly(oxyalkylenes) or poly(ethylene glycol) or polyethylene glycol are especially useful. When the core is small molecularly in nature, any of a variety of hydrophilic functionalities can be used to make the first and second hydrogel precursors water soluble. For example, functional groups like hydroxyl, amine, sulfonate and carboxylate, which are water soluble, maybe used to make the precursor water soluble. In addition, N-hydroxysuccinimide ("NHS") ester of subaric acid is insoluble in water, but by adding a sulfonate group to the succinimide ring, the NHS ester of subaric acid may be made water soluble, without affecting its reactivity towards amine groups.

If it is desired that the biocompatible crosslinked polymer resulting from the reaction of the first and second hydrogel precursors be biodegradable or absorbable, one or more of the first and second hydrogel precursors may have biodegradable linkages present between the functional groups. The biodegradable linkage optionally also may serve as the water soluble core of one or more of the precursors. In the alternative, or in addition, the functional groups of the first and second hydrogel precursors may be chosen such that the product of the reaction between them results in a biodegradable linkage. For each approach, biodegradable linkages may be chosen such that the resulting biodegradable biocompatible crosslinked polymer will degrade, dissolve or be absorbed in a desired period of time. In embodiments, biodegradable linkages may be selected that degrade under physiological conditions into non-toxic products.

The biodegradable linkage may be chelates or chemically or enzymatically hydrolyzable or absorbable. Illustrative chemically hydrolyzable biodegradable linkages include polymers, copolymers and oligomers of glycolide, dl-lactide, 1-lactide, caprolactone, dioxanone, and trimethylene carbonate. Illustrative enzymatically hydrolyzable biodegradable linkages include peptidic linkages cleavable by metalloproteinases and collagenases. Additional illustrative biodegradable linkages include polymers and copolymers of poly(hydroxy acid)s, poly(orthocarbonate)s, poly(anhydride)s, poly(lactone)s, poly(amino acid)s, poly(carbonate)s, poly(saccharide)s and poly(phosphonate)s.

In embodiments, the biodegradable linkage may contain ester linkages. Some non-limiting examples include esters of succinic acid, glutaric acid, propionic acid, adipic acid, or amino acids, as well as carboxymethyl esters.

In embodiments, a multifunctional nucleophilic polymer such as trilysine may be used as a first hydrogel precursor and a multifunctional electrophilic polymer such as a multi-arm PEG functionalized with multiple NHS groups may be used as a second hydrogel precursor. The multi-arm PEG functionalized with multiple NHS groups can for example have four, six or eight arms and have a molecular weight of from about 5,000 to about 25,000. Many other examples of suitable first and second precursors are described in U.S. Patent Nos. 6,152,943; 6,165,201; 6,179,862; 6,514,534; 6,566,406; 6,605,294; 6,673,093; 6,703,047; 6,818,018; 7,009,034; and 7,347,850.

The first hydrogel precursor is applied to a first portion of the porous substrate and a second hydrogel precursor applied to a second portion of the porous substrate. For example, the precursors may be applied in a dry form, such as particulate matter or in a solid or semi-solid state such as a film, or foam. In some embodiments, at least one of the first or second hydrogel precursors may be applied to the porous substrate as a film. The first portion of the substrate having the first hydrogel precursor applied thereto is spatially separated from the second portion of the porous substrate having the second hydrogel precursor applied thereto. Having the first and second hydrogel precursors spatially separated from each other prevents them from reacting with each other until the tape is placed on the wound and exposed to the physiological fluids of a patient.

The first hydrogel precursor may be applied to the porous substrate using any suitable method known to those skilled in the art. For example, the first hydrogel precursor may be incorporated into the porous substrate prior to forming the porous substrate. In another non-limiting example, the first hydrogel precursor may be positioned in the pores of the porous substrate or onto a surface of the porous substrate following formation of the substrate. In additional embodiments, the porous substrate may be calendered prior to application of the first hydrogel precursor thereby allowing the first precursor to penetrate into openings on the substrate which were created by the calendaring process. In still other embodiments, the first hydrogel precursor may be applied to the porous substrate in solution followed by evaporation or lyophilization of the solvent.

The second hydrogel precursor likewise may be applied to the porous substrate using any suitable method known to those skilled in the art. In some embodiments, it is envisioned that a coating may be applied to the substrate in any concentration, dimension and configuration capable of forming a hemostatic tape. In embodiments, the second hydrogel precursor coating may penetrate the pores of the porous substrate. The coating may form a non-porous layer or a porous layer.

In some embodiments, the first hydrogel precursor may be applied to the porous substrate as a solution. In some embodiments, the second hydrogel precursor may be applied to the porous substrate as a solution. The first and second hydrogel precursors may be solubilized in any suitable solvent. Some examples of suitable solvents include, but are not meant to be limited to, water, saline, N-methyl-2-pyrrolidone, 2-pyrrolidone, C₂ to C₆ alkanols, propylene glycol, acetone, alkyl esters such as methyl acetate, ethyl acetate, ethyl lactate, alkyl ketones such as methyl ethyl ketone, dialkylamides such as dimethylformamide, dimethyl sulfoxide, dimethyl sulfone, tetrahydrofuran, cyclic alkyl amides such as caprolactam, decylmethylsufoxide, oleic acid, propylene carbonate, aromatic amides such as N,N-diethyl-m-toluamide, 1-dodecylazacycloheptan-2-one, and the like. Once the first and/or second hydrogel precursors solutions are applied to the porous substrate, the solvent may be removed from the porous substrate, leaving the first and/or second hydrogel precursor positioned in the porous substrate in a particle or dehydrated form. The solvent may be removed using any method suitable for drying or driving-off a solvent.

The hemostatic tapes described herein are self-adhering when placed in contact with physiologic fluids, such as, sweat, blood, mucous, saliva, and the like, as well as any water and saline. The tapes adhere to the wound and/or the surrounding skin after the physiologic fluids interact with the first and second hydrogel precursors. In embodiments, the fluids dissolve and mix the first and second hydrogel precursors as the fluids pass through the porous substrate.

In other embodiments, the tapes may include barbed fibers or filaments. The barbs may be added to any portion of the tape. The barbs may enhance the tapes ability to adhere to tissue. It is envisioned that the barbs may also enhance the tapes ability to adhere to other portions of the tape when wrapped about tissue. For instance, the barbed tape may be wrapped around an organ so that a first portion of the barbed tape overlaps a second portion of the barbed tape and the barbs on the first portion may adhere to the second portion of the tape.

Although the tapes may be self-adherent when in contact with physiologic fluids, in some embodiments the tapes may further include an adhesive layer. In some embodiments, the adhesive layer may be positioned on a surface of the porous substrate which faces the wound to provide the tape with an initial adhesive property prior to the interaction of a physiologoic fluid with the first and second hydrogel precursors in the tape. In other embodiments, an adhesive layer may be positioned on a surface of the porous substrate which faces away from the wound to attach a support layer to the tape. In still other embodiments, the tapes described herein may include more than one adhesive layer. The adhesive layer may be applied to a portion of the tape in a continuous or discontinuous manner and may represent any suitable pattern or concentration.

The adhesive layer includes any adhesive material suitable for adhering to the porous substrate. The adhesive material is not particularly limited to any specific material. However, in some embodiments, the adhesive layer includes a pressure-sensitive adhesive suitable for medical use. Some non-limiting examples include acrylic-based adhesives, rubber-based adhesives, silicone-based adhesives, and blends of two or more of them.

In some embodiments, the adhesive layer may be formed on a surface of the porous substrate as a continuous film having a thickness of about 10 to about 200 µm. In other embodiments, the adhesive layer may be formed on a surface of the porous substrate as a discontinuous film displaying any pattern, shape or thickness suitable for adhering to the porous substrate.

In addition to the porous substrate, the hemostatic tapes described herein include a release sheet positioned over at least a portion of the porous substrate. The release sheet may be designed to prevent contact or interaction with the porous substrate which includes a first and second hydrogel precursor prior to application to a wound thereby allowing a person to handle the tape without fear of prematurely attaching the tape to an unintended surface. The release sheet may be removably attached to at least one surface of the porous substrate. The release sheet also allows the tape to be folded, rolled or twisted in any manner while preventing one part of the porous substrate from contacting another part of the porous substrate thereby preventing the tape from sticking to itself. The release sheet may be made from any bioabsorbable or non-bioabsorbable material which is non-reactive to the porous substrate, the first hydrogel precursor and the second hydrogel precursor. Some examples include, but are not limited to, polylactones, polytetrafluoroethylene, polyvinyl chloride, polyolefins, metalized polymer films, metallic foils, silicone-based materials, polyvinyl pyrrolidones, polyethylene glycols, polyvinyl alcohols, polyacrylic acids, carboxymethylcellulose, alginates, hyaluronic acids, dextrans, polysaccharides, gelatins and combinations thereof.

The release sheet may be removably attached to the porous substrate. By removable, the release sheet may be positioned and/or repositioned on or off the porous substrate as needed. The release sheet may be removably positionable. In some embodiments, the release sheet may be formed prior to being attached to the porous substrate. For example, the release sheet may be a paper film coated with silicone which is shaped to size prior to being removably attached to the porous substrate. In other embodiments, the release sheet may be formed while in contact with the porous substrate.

The release sheet may be positioned on at least one surface of the porous substrate to prevent the porous substrate from coming in contact with water, saline, moisture or other physiologic fluids. In some embodiments, the porous substrate may be positioned between a top release sheet and a bottom release sheet. In other embodiments, the porous substrate may be sealed within a release sheet which encapsulates all sides of the porous substrate and prevents moisture and fluids from reaching the porous substrate. It is envisioned that the release sheet may be formed to accommodate any shape, or size porous substrate. In still other embodiments, a release sheet may be positioned on top of a backing sheet.

A backing sheet may be applied to the surface of the porous substrate which faces away from the wound. In general, the backing sheet may be made from any material, woven or non-woven, synthetic or natural, porous or non-porous, perforated or non-perforated, elastic or non-elastic, which will provide support and may also act as a protective covering for the porous substrate after being applied to the wound. Some non-limiting examples of suitable materials include, for example, cellophane, cellulose acetate, ethyl cellulose, plasticized vinyl acetate-vinyl chloride copolymers, ethylenevinyl acetate copolymer, polylactide, polycaprolactone, polyglycolide, polytrimethylene carbonate, polyethylene terephthalate, nylon, polyethylene, polypropylene, polyurethane, polyvinylidene, chloride, biopolymers, such as collagen, elastin, dextran, alginate, chitosan, keratin, and combinations thereof. In embodiments, the backing sheet may be in the form of a film. In other embodiments, the backing sheet may be a fabric. The backing sheet may be impermeable to moisture vapor, liquids, gas and/or bacterial growth. The backing sheet may be opaque or transparent. The width and thickness of the backing sheet will vary according to the size and shape of the porous substrate employed.

The backing sheet may be fixedly attached to the porous substrate. In some embodiments, the backing sheet may be formed prior to being attached to the porous substrate. In other embodiments, the backing sheet may be formed while in contact with the porous substrate. In some embodiments, an adhesive may be used to fixedly attach a backing sheet to the porous substrate.

In addition to providing tissue support and hemostasis, the tapes may further be used for delivery of a bioactive agent. Thus, in some embodiments, at least one bioactive agent may be combined with any part of the tape including the porous substrate, the first hydrogel precursor, the second hydrogel precursor, the backing material, the release sheet, the adhesive layer and/or may be separately applied to the tape. The agents may be freely admixed with the precursors or may be tethered to the precursors through any variety of chemical bonds. In these embodiments, the present tape may also serve as a vehicle for delivery of the bioactive agent.

The term "bioactive agent", as used herein, is used in its broadest sense and includes any substance or mixture of substances that have clinical use. Consequently, bioactive agents may or may not have pharmacological activity per se, e.g., a dye, or fragrance. Alternatively a bioactive agent could be any agent which provides a therapeutic or prophylactic effect, a compound that affects or participates in tissue growth, cell growth, cell differentiation, an anti-adhesive compound, a compound that may be able to invoke a biological action such as an immune response, or could play any other role in one or more biological processes. It is envisioned that the bioactive agent may be applied to the present tape in any suitable form of matter, e.g., films, powders, liquids, gels and the like.

Examples of classes of bioactive agents which may be utilized in accordance with the present disclosure include anti-adhesives, antimicrobials, analgesics, antipyretics, anesthetics, antiepileptics, antihistamines, anti-inflammatories, cardiovascular drugs, diagnostic agents, sympathomimetics, cholinomimetics, antimuscarinics, antispasmodics, hormones, growth factors, muscle relaxants, adrenergic neuron blockers, antineoplastics, immunogenic agents, immunosuppressants, gastrointestinal drugs, diuretics, steroids, lipids, lipopolysaccharides, polysaccharides, platelet activating drugs, clotting factors and enzymes. It is also intended that combinations of bioactive agents may be used.

Suitable antimicrobial agents which may be included as a bioactive agent in the bioactive coating of the present disclosure include triclosan, also known as 2,4,4'-trichloro-2'-hydroxydiphenyl ether, chlorhexidine and its salts, including chlorhexidine acetate, chlorhexidine gluconate, chlorhexidine hydrochloride, and chlorhexidine sulfate, silver and its salts, including silver acetate, silver benzoate, silver carbonate, silver citrate, silver iodate, silver iodide, silver lactate, silver laurate, silver nitrate, silver oxide, silver palmitate, silver protein, and silver sulfadiazine, polymyxin, tetracycline, aminoglycosides, such as tobramycin and gentamicin, rifampicin, bacitracin, neomycin, chloramphenicol, miconazole, quinolones such as oxolinic acid, norfloxacin, nalidixic acid, pefloxacin, enoxacin and ciprofloxacin, penicillins such as oxacillin and pipracil, nonoxynol 9, fusidic acid, cephalosporins, and combinations thereof. In addition, antimicrobial proteins and peptides such as bovine lactoferrin and lactoferricin B may be included as a bioactive agent suitable for delivery by the tapes of the present disclosure.

Other bioactive agents which may be applied to the tapes in accordance with the present disclosure include: local anesthetics; non-steroidal antifertility agents; parasympathomimetic agents; psychotherapeutic agents; tranquilizers; decongestants; sedative hypnotics; steroids; sulfonamides; sympathomimetic agents; vaccines; vitamins; antimalarials; anti-migraine agents; anti-parkinson agents such as L-dopa; antispasmodics; anticholinergic agents (e.g. oxybutynin); antitussives; bronchodilators; cardiovascular agents such as coronary vasodilators and nitroglycerin; alkaloids; analgesics; narcotics such as codeine, dihydrocodeinone, meperidine, morphine and the like; non-narcotics such as salicylates, aspirin, acetaminophen, d-propoxyphene and the like; opioid receptor antagonists, such as naltrexone and naloxone; anti-cancer agents; anti-convulsants; anti-emetics; antihistamines; anti-inflammatory agents such as hormonal agents, hydrocortisone, prednisolone, prednisone, non-hormonal agents, allopurinol, indomethacin, phenylbutazone and the like; prostaglandins and cytotoxic drugs; chemotherapeutics, estrogens; antibacterials; antibiotics; anti-fungals; anti-virals; anticoagulants; anticonvulsants; antidepressants; antihistamines; and immunological agents.

Other examples of suitable bioactive agents which may be included in the tape include viruses and cells; peptides; polypeptides and proteins, analogs, muteins, and active fragments thereof, such as immunoglobulins, antibodies, cytokines (e.g. lymphokines, monokines, chemokines); blood clotting factors; hemopoietic factors; interleukins (IL-2, IL-3, IL-4, IL-6); interferons (β-IFN, α-IFN and γ-IFN); erythropoietin; nucleases; tumor necrosis factor; colony stimulating factors (e.g., GCSF, GM-CSF, MCSF); insulin; anti-tumor agents and tumor suppressors; blood proteins; fibrin; thrombin; fibrinogen; synthetic thrombin; synthetic fibrin; synthetic fibrinogen; gonadotropins (e.g., FSH, LH, CG, etc.); hormones and hormone analogs (e.g., growth hormone); vaccines (e.g., tumoral, bacterial and viral antigens); somatostatin; antigens; blood coagulation factors; growth factors (e.g., nerve growth factor, insulin-like growth factor); bone morphogenic proteins, TGF-B, protein inhibitors, protein antagonists, and protein agonists; nucleic acids, such as antisense molecules, DNA, RNA, RNAi; oligonucleotides; polynucleotides; and ribozymes.

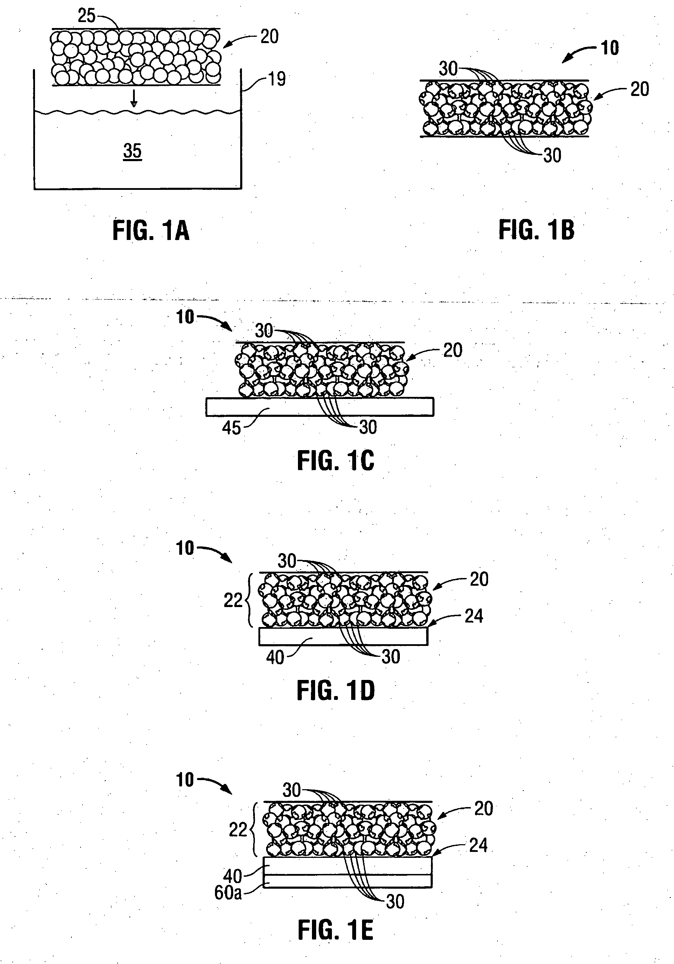

Turning now to Figures 1A - E, a sequence is shown wherein a first hydrogel precursor is applied within the pores of a porous substrate and a second hydrogel precursor is applied to a second portion of the porous substrate. In Fig. 1A, porous substrate 20 is a foam having a plurality of pores 25 defined therein. Solution 35, which includes a first hydrogel precursor dissolved in a solvent, is stored in container 19. Porous substrate 20 is dipped into and completely submerged within solution 35. Upon removal, the substrate is dried, removing the solvent from solution 35 and depositing particles that include the first hydrogel precursor 30 within pores 25 of substrate 20, as shown in Fig. 1B.

In Fig. 1C, porous substrate 20 containing the first hydrogel precursor is contacted with a melt 45 of the second hydrogel precursor. Upon cooling, the melt 45 of the second hydrogel precursor will solidify to form a film 40 over at least a portion of substrate 20. After application of the film 40 of the second precursor, the tape may be trimmed to any desired size and shape. Tape 10 of Fig. 1D is shown having a first hydrogel precursor in the form of particles 30 applied to first portion 22 of porous substrate 20 and a second hydrogel precursor in the form of a film 40 applied to second portion 24 of porous substrate 20.

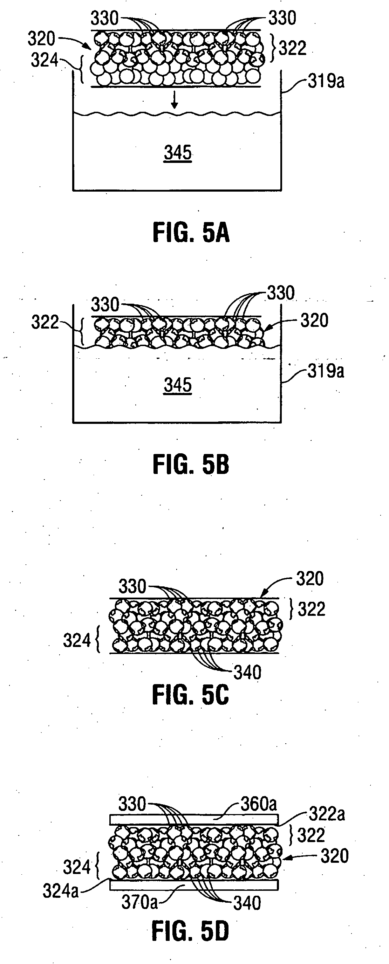

In Fig. 1E, a first release sheet 60a is removably attached to a portion of tape 10. First release sheet 60a is shown removably attached to film 40 of tape 10. In some embodiments, a first release sheet may be removably attached to the porous substrate portion of the tape. In some embodiments, a second release sheet may be removably attached to a different portion of the tape (see Fig. 5D).

In some embodiments, an adhesive may be applied to the release sheet prior to being removably attached to a portion of the tape. In some embodiments, the release sheet may be removably attached to the tape while the release sheet is being formed.

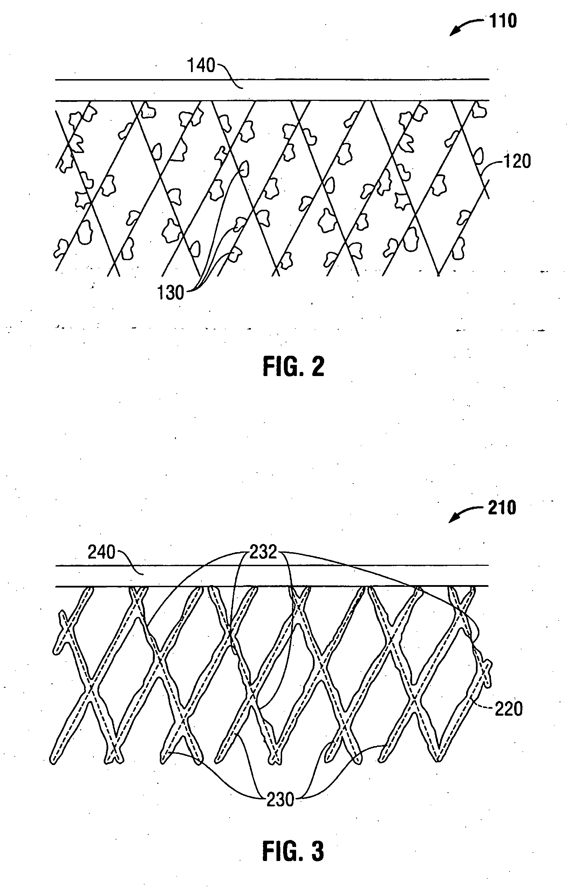

Tape 110 of Fig. 2 may be prepared in a manner similar to that shown in the sequence of Figures 1A-E, with the exception that the porous substrate 120 is a mesh material having a first hydrogel precursor in the form of particles 130 and a second hydrogel precursor in the form of a film 140 applied thereto. It is contemplated that a non-woven material (not shown) may be used as the porous substrate instead of the foam shown in Figures 1A-E or the mesh shown in Figure 2.

Tape 210 of Fig. 3 may be prepared in a manner similar to that shown in the sequence of Figures 1A-E, with the exception that the porous substrate 220 is a mesh material having a first hydrogel precursor in the form of a coating 230 and a second hydrogel precursor in the form of a film 240 applied thereto. Coating 230 of the first hydrogel precursor may be formed by immersing porous substrate 220 into a solution of the first hydrogel precursor or into a melt of the first hydrogel precursor. Alternatively, the first hydrogel precursor may be combined with a film-forming polymer prior to application to the substrate to provide coating 230. Those skilled in the art reading this disclosure will envision other method and materials for applying a coating containing the first hydrogel precursor to the substrate.

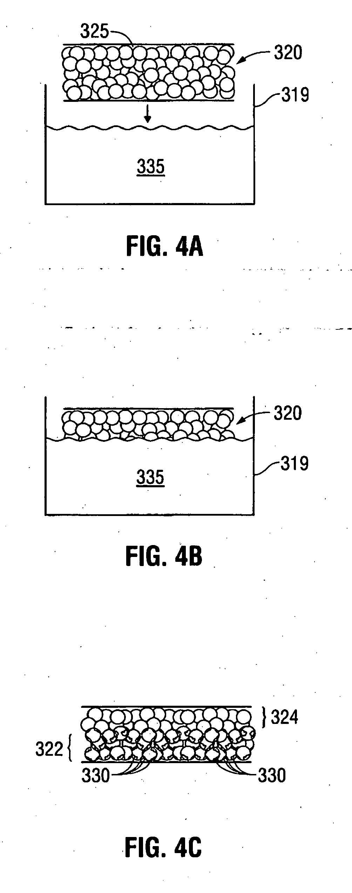

Turning now to Figs. 4A-4C, a sequence is shown wherein a first hydrogel precursor may be applied to a first portion of a porous substrate. In Fig. 4A, porous substrate 320 may be a foam material having a plurality of pores 325 defined therein, which includes at least a first portion 322 and a second portion 324. Solution 335, which includes a first hydrogel precursor dissolved in a solvent, may be stored in container 319. Porous substrate 320 may be positioned over solution 335 with first portion 322 facing solution 335 and second portion 324 facing away from solution 335.

In Fig. 4B, first portion 322 of porous substrate 320 is partially submerged in solution 335 by moving porous substrate 320 in the direction of solution 335, as represented by the arrow in Fig. 4A. Only first portion 322 of porous substrate 320 comes in contact with solution 335 so that a sufficient amount of solution 335 may be applied to and fill the pores 325 of first portion 322 of porous substrate 320. Upon removal, the tape is dried, removing the solvent from solution 335 and depositing particles that include the first hydrogel precursor 330 in first portion 322, as shown in Fig. 4C. Particles 330 include the first hydrogel precursor in a dry format and are limited spatially to first portion 322.

In Figs. 5A-5D, a sequence is shown wherein solution 345 containing a second hydrogel precursor dissolved in a solvent is applied to second portion 324 of porous substrate 320, wherein particles 330 containing a first hydrogel precursor have been previously incorporated into first portion 322 of substrate 320 (See Figs. 4A-4C). Porous substrate 320 is positioned over solution 345 with second portion 324 facing solution 345 and first portion 322 facing away from solution 345.

As shown in Fig. 5B, second portion 324 of porous substrate 320 is partially submerged in solution 345 by moving porous substrate 320 in the direction of solution 345, as represented by the arrow in Fig. 5A. Only second portion 324 of porous substrate 320 comes in contact with solution 345 so that a sufficient amount of solution 345 may be applied to second portion 324. Upon removal, the tape is dried to deposit second particles 340 including the second hydrogel precursor in second portion 324. Particles 340 include the second hydrogel precursor in a dry format and are limited spatially to second portion 324. Porous substrate 320 of Fig. 5C is shown having a first hydrogel precursor applied to a first portion of the substrate and a second hydrogel precursor applied to a second portion of the porous substrate with the first portion of the substrate being spatially separated from the second portion of the porous substrate.

In Fig. 5D a release sheet 360a is removably attached to a first surface 322a of porous substrate 320. In addition, an adhesive may be applied to second surface 324a to fixedly attach backing sheet 370a to porous substrate 320.

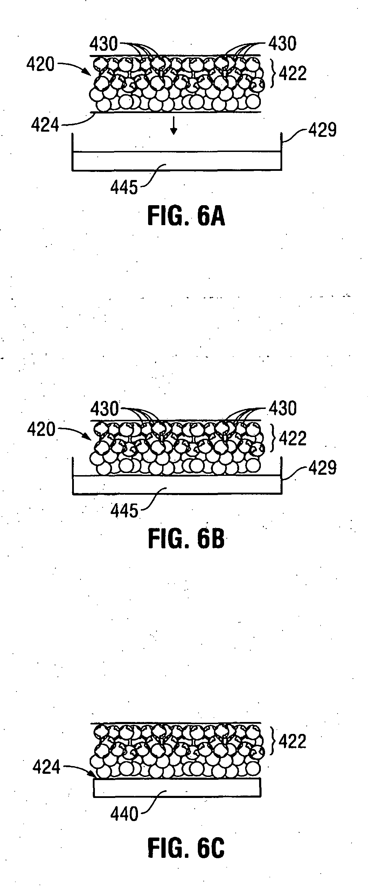

In alternative embodiments, the first and second hydrogel precursors may be applied to the tape in different forms. For example, in Figs. 6A-6C, porous substrate is shown including particles 430 including the first hydrogel precursor applied to first portion 422 with second portion 424 facing a film-forming solution 445A containing the second hydrogel precursor that has been applied to a support 429.

In Fig. 6B, second portion 424 of porous substrate 420 is contacted with and/or partially submerged in film-forming solution 445 by moving porous substrate 420 in the direction of shown by the arrow in Fig. 6A. Only second portion 424 of porous substrate 420 comes in contact with film-forming solution 445 so that a sufficient amount of material 445 may be applied to second portion 424. Film-forming solution 445 is allowed solidify (with or without the application of heat) to form a film over at least a portion of second portion 424. Porous substrate 420 of Fig. 6C is shown having a first hydrogel precursor in the form of particles applied to a first portion of the substrate and a second hydrogel precursor in the form of a film applied to a second portion of the porous substrate with the first portion of the substrate being spatially separated from the second portion of the porous substrate.

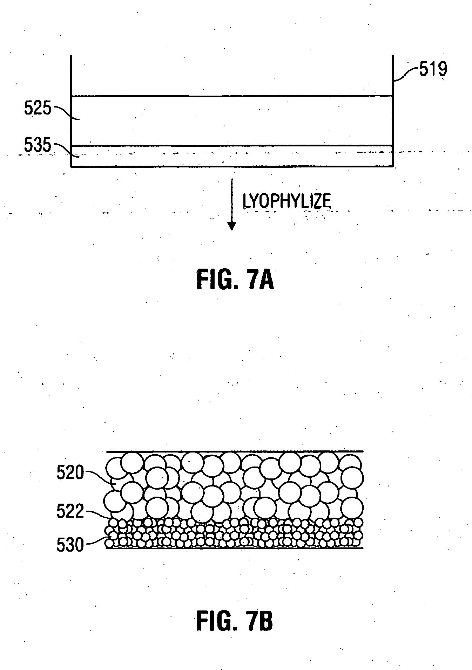

Turning now to Figs. 7A-7B, the porous substrate and a porous layer including the first hydrogel precursor are shown formed together. In Fig. 7A, container 519 includes first solution 525 destined to form the porous substrate and a second solution 535 including the first hydrogel precursor, wherein the two solutions remain substantially as separate layers. The two solutions are lyophilized using any method known to those skilled in the art to form a porous substrate as shown in Fig. 7B, which includes first porous substrate 520, made from the lyophilized first solution 525, connected to a second porous layer 530, made from the lyophilized second solution 535. Second porous layer 530 contains the first hydrogel precursor and is bonded to first porous substrate 520 via first portion 522 to form an tape having two layers of porous material.

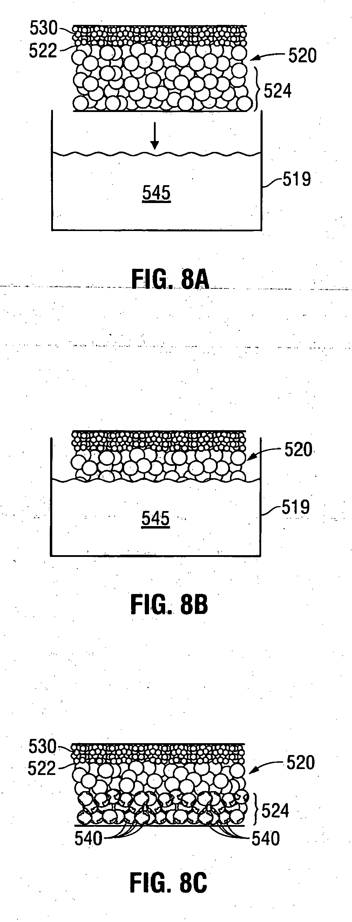

In Figs. 8A-8C, a sequence is shown wherein solution 545 containing a second hydrogel precursor is applied to second portion 524 of porous substrate 520 already having porous substrate 530 including the first hydrogel precursor bonded thereto porous at first portion 522. Porous substrate 520 is positioned over solution 545 with second portion 524 facing solution 545 and first portion 522 and second porous layer 530 facing away from solution 545.

As shown in Fig. 8B, second portion 524 of porous substrate 520 is partially submerged in solution 545 having the first hydrogel precursor dissolved in a solvent by moving porous substrate 520 in the direction of solution 545, as represented by the arrow in Fig. 8A. Only second portion 524 of porous substrate 520 comes in contact with solution 545 so that a sufficient amount of solution 545 may be applied to second portion 524. Upon removal, the tape is dried or allowed to dry to remove the solvent and deposit particles 540 in second portion 524. Second particles 540 include the second hydrogel precursor in a dry format and are limited spatially to second portion 524. Porous substrate 520 of Fig. 8C is shown having a first hydrogel precursor in the form of a foam applied to a first portion of the substrate and a second hydrogel precursor in the form of particles applied to a second portion of the porous substrate with the first portion of the substrate being spatially separated from the second portion of the porous substrate.

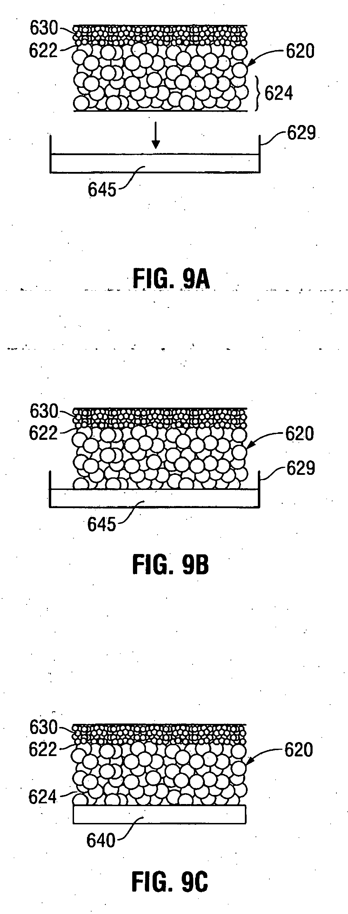

In an alternative embodiment, the porous substrate as shown in Fig. 7B may be combined with a film-forming material including the second hydrogel precursor. As shown in Figs. 9A-9C, porous substrate 620 includes a first portion 622 and a second portion 624, wherein a second porous layer 630 containing a first hydrogel precursor is connected to porous substrate 620 at first portion 622. Second portion 624 is shown facing a film-forming solution 645 applied to support 629. Film-forming material 645 includes a second hydrogel precursor and a solvent.

In Fig. 9B, second portion 624 of porous substrate 620 is contacted with and/or partially submerged in film-forming solution 645 by moving porous substrate 620 in the direction of represented by the arrow in Fig. 9A. Only second portion 624 of porous substrate 620 comes in contact with film-forming solution 645 so that a sufficient amount of material 645 may be applied to second portion 624. Film-forming solution 645 is allowed to form a film over at least a portion of second portion 624. Porous substrate 620 of Fig. 9C is shown having a first hydrogel precursor in the form of a foam applied to a first portion of the substrate and a second hydrogel precursor in the form of a film applied to a second portion of the porous substrate with the first portion of the substrate being spatially separated from the second portion of the porous substrate.

It should be understood that the tapes as shown in Figures 6-9, may be removably attached to any number of release sheets and fixedly attached to a backing sheet as provided previously in Figures 1-5. Any combination of backing sheets and release sheets may be utilized in forming the tapes described herein.

It should be further understood that rather than a foam, as shown in Figures 1, and 4 - 9, the porous substrate may be a fibrous structure, i.e., a woven, knitted, or non-woven structure (see Figures 2 and 3). The first and second hydrogel precursors can be applied to a fibrous porous substrate using substantially the same techniques described above with respect to foam porous substrate 20. The release sheet and/or backing sheets may also be attached to a fibrous porous substrate using substantially the same techniques described above with respect to foam porous substrate 20. Accordingly, as with the foam porous substrates described above, where the porous substrate is fibrous, the release sheet with prevent the fibrous structure from adhering to an unwanted surface prematurely and a backing sheet will provide further support and protection to the self-adhering fibrous structure.

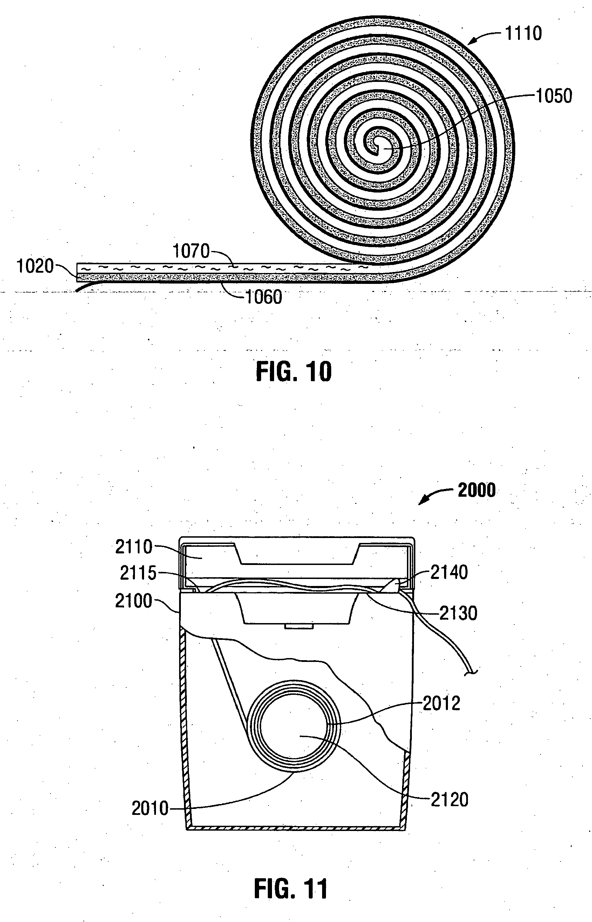

Turning now to Fig. 10, tape 1110 is shown in a roll configuration on spool 1050. First hydrogel precursor 1030 and second hydrogel precursor 1040 are applied to porous substrate 1020 utilizing any of the previously described methods. Backing sheet 1070 is attached to a first side of porous substrate 1020. Release sheet 1060 is removably attached to a second side of porous substrate 1020. In a roll configuration, release sheet 1060 prevents neighboring layers of porous substrate 1020 and backing sheet 1070 from interacting. Backing sheet 1070 and release sheet 1060 may be made of materials which do not interact with one another thereby maintaining the ability of release sheet 1060 to be removably attached from substrate 1020.

Turning now to FIG. 11 a hemostatic tape dispenser is illustrated in accordance with the present disclosure, generally designated as 2000. Dispenser 2000 includes housing 2100 which may be configured to store and dispense tape 2010 in a roll configuration. Housing 2100 may include cap 2110, first support member 2120, second support member 2130 and at least one cutting member 2140. Tape 2010 is in a roll configuration wrapped about spool 2012. Spool 2012 is positioned about first support member 2130 in housing 2100. In other embodiments, tape 2010 may be in a roll configuration wrapped about first support member 2130 directly.

Housing 2100 of dispenser 2000 may be made of any material. In some embodiments, housing 2100 is molded from a suitable plastics material, as will be evident to persons skilled in the art; and cutting member 2140 may be formed from metal, usually by stamping is made is plastic. In some embodiments, housing 2100 is made of one or more individually molded pieces or may be co-molded as one piece.

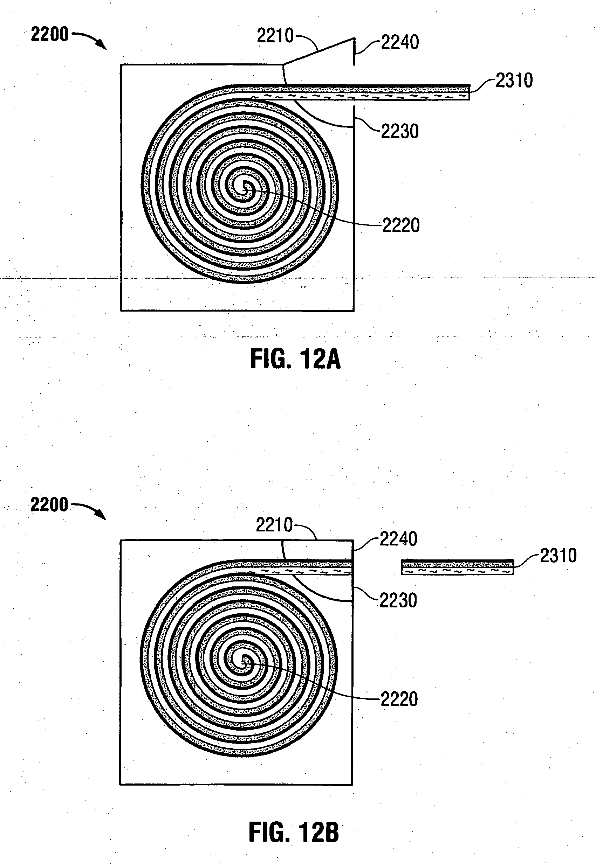

Cap 2110 may be pivotably connected to housing 2100 and may be designed to pivot from an open position (FIG. 12A) to a closed position (FIG. 12B). In the closed position, cap 2110 prevents moisture and liquids from penetrating housing 2100. Cap 2110 may pivot about housing 2100 in any manner known to those skilled in the art. In some embodiments, cap 2110 may be a flip-top lid, which may be connected to any side of housing 2100 via hinge 2115.

First support member 2120 may be positioned on housing 2100. First support member 2120 may be used to support tape 2010 in a roll configuration. Second support member 2130 may also be positioned on housing 2100. Second support member 2130 may be configured to receive a length of tape 2010 which extends from the roll. It is envisioned that the housing may include any number of support members as needed to properly store and dispense the hemostatic tape. It is further envisioned that support members may be positioned along any portion of the housing suitable for extending a length of tape between the support members.

Housing 2100 also includes cutting member 2140. Cutting member 2140 is operatively coupled with housing 2100 and configured to separate at least a portion of the length of tape 2010 from the roll. Cutting member 2140 is a sharpened or serrated edge capable of separating the tape into any variety of smaller sections. Cutting member 2140 may be co-formed with the housing 2100 or formed separate therefrom. In some embodiments, a length of tape may be perforated for ease of cutting or separation.

In some embodiments, cutting member 2140 is coupled to second support member 2130. In such embodiments, a portion of the length of tape 2010 received by second support member 2130 may be forced against cutting member 2140 to separate the length of tape 2010 from the roll.

In other embodiments, as shown in Figs. 12A-B, at least one cutting member 2240 is coupled to cap 2210. In such embodiments, cap 2210 may be moved from an open position (Fig. 12 A) to a closed position (Fig. 12B) to force a portion of the length of tape 2310 received by second support member 2230 against cutting member 2240 and separate the length of tape 2310b from the roll. Roll of tape 2310 is positioned on first support member 2220. In embodiments, cutting member 2240 may be positioned on both housing 2200 and cap 2210 and may be designed to engage one another when forced into the closed position. As shown in Fig. 12B, tape 2310 is sealed within dispenser 2200 by cap 2210 to prevent tape 2310 from being prematurely exposed to moisture or other fluids.

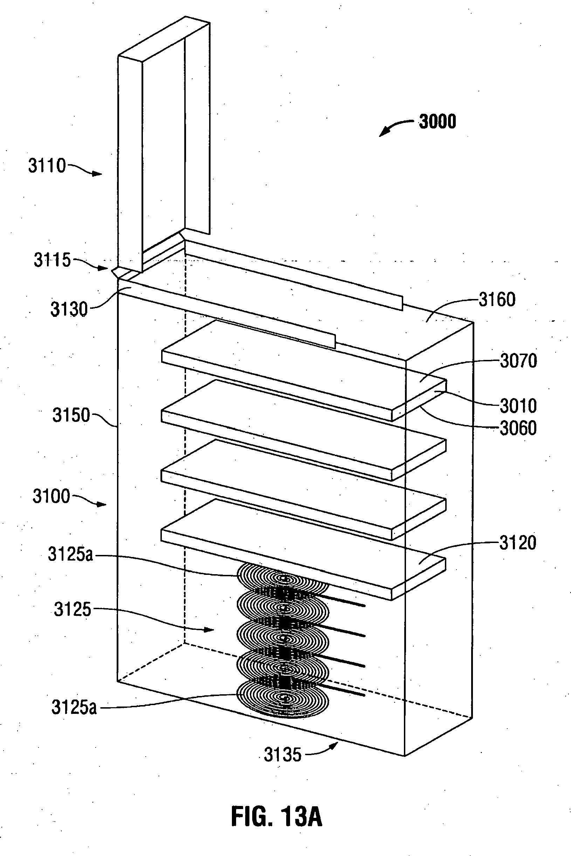

As shown in Figs. 13A-C, hemostatic tape dispenser 3000 includes housing 3100, which is be configured to store and dispense tape 3010 in a stacked configuration. The housing 3100 may include a base 3135, at least one side wall 3150 extending from base 3135, cap 3110, biasing member 3125, retaining member 3130, first support member 3120 and opening 3160. The at least one side wall 3150 extends proximally from base 3135 to form a cavity suitable for receiving tape 3010 in a stacked or folded configuration.

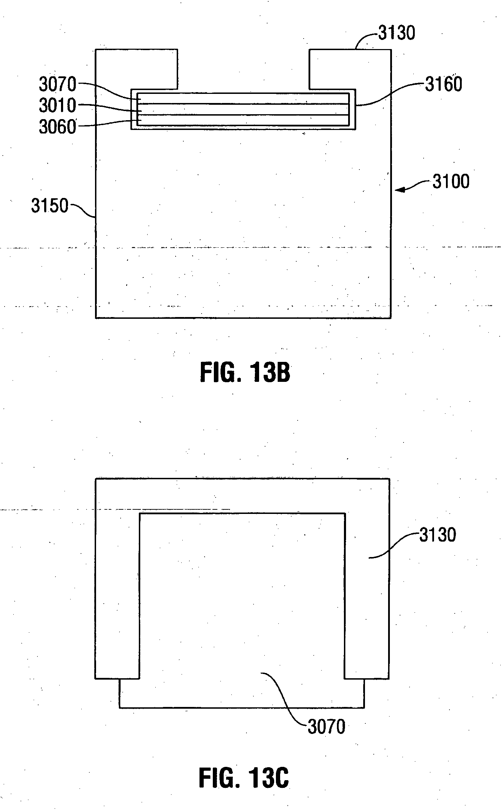

A plurality of individual strips of tape 3010 may be stacked between first support member 3120 and retaining member 3130. Backing sheet 3070 covers the top portion of hemostatic tape 3010. Release sheet 3060 covers the bottom of the tape 3010. As shown in Figs. 13B and 13C, retaining member 3130 is positioned near the proximal end of side wall 3150 of housing 3100 and extends inwardly into the cavity thereby providing an edge around a portion of the perimeter of side wall. The retaining member prevents tape 3010 from escaping proximally through the top of housing 3100.

Biasing member 3125, e.g., a spring, is positioned between base 3135 and first support member 3120 with distal end 3125b of biasing member 3125 positioned near base 3135 and proximal end 3125a of biasing member 3125 positioned near first support member 3120. Biasing member 3125 is designed to move first support member 3120 away from base 3135 forcing tape 3010 towards retaining member 3130 and opening 3160.

Opening 3160 is defined within a portion of side wall 3150 which is distal to retaining member 3130. Opening 3160 is configured to allow tape 3010 to be withdrawn laterally from housing 3100. It is envisioned that the removal of an individual strip of tape 3010 from housing 3100 allows biasing member 3125 to move first support member 3120 proximally towards retaining member 3130 thereby positioning the next strip of hemostatic tape 3010 in abutting relationship with retaining member 3130 and at opening 3160.

Cap 3110 may be pivotably connected to housing 3100 via hinge 3115. Cap 3110 may be designed to go from an open position (shown) to a closed position (not shown). Cap 3110 may be configured to cover retaining member 3130 and opening 3160 thereby sealing tape 3010 within housing 3100.

A user may place his or her finger along the opening and draw the hemostatic tape 3010 laterally from the dispenser 3000. Backing tape 3070 protects tape 3010 as it is pulled from dispenser 3000, while release sheet 3060 may be removed by a user prior to application to a wound.

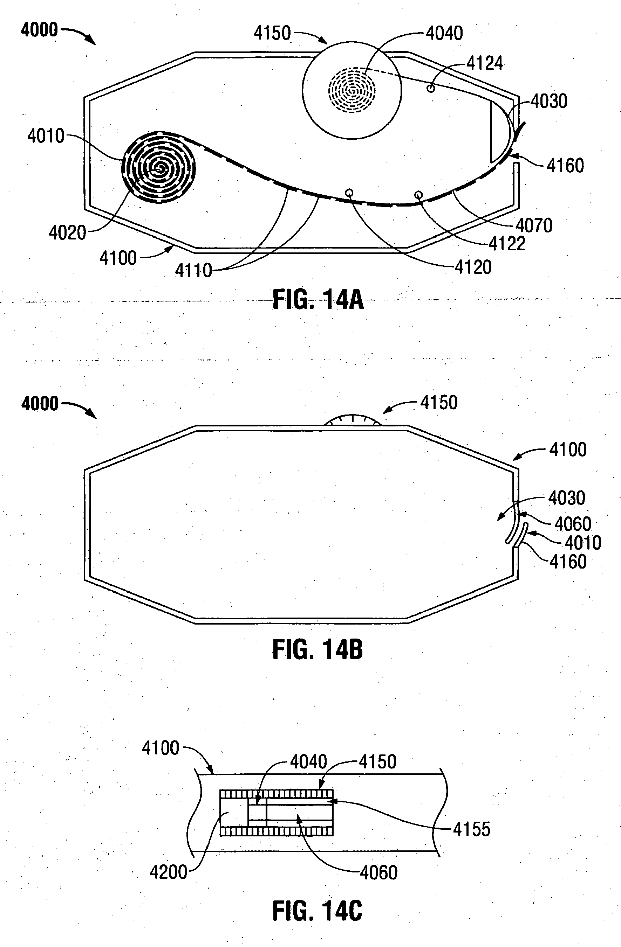

Another embodiment of a dispenser is shown in Figs. 14A-C. Dispenser 4000 includes housing 4100, first support member 4020, a plurality of guide pins 4120, 4122, 4124, second support member 4030, third support member 4040, slot 4060, and wheel member 4150. A roll of tape 4010, including backing sheet 4070, is positioned on first support member 4020. A length of tape 4010 extends between first support member 4020 and third support member 4040 which is connected to wheel member 4150.

Wheel member 4150, e.g., a thumb wheel, may be turned to draw tape 4010 from first support member 4020 around guide pins 4120, 4122 towards second support member 4030 which extends slightly through slot 4160. At slot 4160, tape 4010 is separated from backing sheet 4060 and withdrawn from housing 4100 while backing sheet 4060 continues without the hemostatic tape to be drawn around guide pin 4124 and onto third support member 4040.

As shown in Fig 14B, at least a portion of wheel member 4150 and second support member 4030 with backing sheet 4060 are visible from outside the dispenser. As wheel member 4150 is turned, backing sheet 4060 is pulled around the second support member 4030 and tape 4010 is exposed for removal from the device 4000.

In some embodiments, the dispenser may include a window which allows a user to see at least a portion of the tape stored within the housing. The window may be positioned on any portion of the housing. As shown in Fig. 14C, window 4200 is positioned near wheel member 4150 to allow a user to see backing sheet 4060 as it is pulled onto third support member 4040. In embodiments, backing sheet 4060 may be colored as the end of the backing sheet 4060 is reached so that a user knows when the tape is running out.

In embodiments, these dispensers may be single use, or refillable. In some embodiments, the housing may also include a desiccant (not shown). The desiccant may be made from any material suitable for absorbing moisture. For example, the desiccant may include silica gel, zeolites, aluminas, calcium sulfate, calcium chloride, montmorillonite clay, or molecular sieves.

During use, a length of the hemostatic tape may be pulled from the dispenser and separated from the roll by the cutting member of the dispenser. In embodiments, the individually pre-cut tape segments may be removed as described above. The release sheet may be removed from the hemostatic tape. The hemostatic tape may be placed on a wound with the first hydrogel precursor facing the wound. Upon contacting the wound, physiologic fluids near the wound will enter the porous substrate portion of the hemostatic tape thereby dissolving the first hydrogel precursor. The fluid wicks and/or migrates across the hemostatic tape carrying the first hydrogel precursor through the porous substrate. When the fluid reaches the second hydrogel precursor, the second hydrogel precursor will dissolve and interact with the first hydrogel precursor forming a biocompatible cross-linked material which adheres the tape to the tissue and provides hemostasis to the wound.

Upon application to a wound, the hemostatic tape may affect hemostasis of the wound. As used herein, the term "hemostasis" means the arrest or cessation of bleeding. Hemostasis may occur, at the site of application of the hemostatic tape, within less than about 120 seconds. In some embodiments, hemostasis may occur within less than about 15 seconds. In yet another embodiment, hemostasis may occur within less than about 5 seconds. In embodiments, an adhesive may positioned along a portion of the tape which will come in contact with the wound to provide immediate adherence to the tissue alllowing the tape time to absorb the wound fluids and mix the first and hydrogel precursors.

### EXAMPLE

A saturated borate buffer solution of trilysine is prepared. The solution contains 20.6 milligrams of trilysine per milliliter of solution. The pH of the solution is about 9.2. A porous sheet of oxidized cellulose is dipped into the solution and then fixed to a rack for drying. The rack is placed into a vacuum oven. The oven is pumped down to about 50 mTorr and kept at a temperature of about 25°C for about three days to reduce the moisture level to less than 2% by weight. An eight arm N-hydroxysuccinimidyl-functionalized polyethylene glycol having a molecular weight of about fifteen thousand is melted at about 50° C. on a hot plate. The dried trilysine-containing oxidized cellulose sheet is placed into contact with the melted PEG component. After cooling, the PEG component forms a film on one side of the porous sheet.

A first release sheet and a second release sheet made from paper and coated with a silicone material are positioned on the porous sheet. The first release sheet is positioned on the film side of the porous sheet. The second release sheet is positioned on the side opposite the film on the porous substrate.

The resulting product is trimmed into long strips with a width of about 0.5cm and a length of about 75 cm, dried, and rolled onto a spool. The rolled tape is sterilized and packaged in a sealed dispenser.

In use, the sealed dispenser is opened and a portion of the tape is dispensed from the container. The release sheet is removed from the tape and the tape is applied to a bleeding wound with the PEG film side against the wound. Within seconds, the tape is adhered to the tissue and hemostasis occurs.

## Claims

1. A hemostatic tape comprising:
a porous substrate (20, 120, 220, 320, 420, 520, 620) having a fibrous or a foam structure;
a first hydrogel precursor (30, 130, 230, 330, 430, 530, 630) and a second hydrogel precursor (40, 140, 240, 340, 440, 540, 540, 640) applied to the porous
substrate; and
a release sheet (60a, 360a)disposed on at least one side of the porous substrate,
**characterised in that** the first hydrogel precursor is applied to a first portion (22, 322, 422, 522, 622) of the porous substrate and the second hydrogel precursor is applied to a second portion (24, 324, 424, 524, 624) of the porous substrate.

2. The hemostatic tape of claim 1 wherein the porous substrate is a foam.

3. The hemostatic tape of claim 1 wherein the porous substrate is made from a fibrous material.

4. The hemostatic tape of claim 1 wherein the porous substrate is made from oxidized cellulose.

5. The hemostatic tape of any preceding claim wherein the first hydrogel precursor is selected from the group consisting of particles, a foam, a film and combinations thereof.

6. The hemostatic tape of any preceding claim wherein the second hydrogel precursor is selected from the group consisting of particles, a foam, a film and combinations thereof.

7. The hemostatic tape of any preceding claim wherein the release sheet is made from a silicone-based material.

8. The hemostatic tape of any preceding claim further comprising a backing sheet (370a).

9. The hemostatic tape of any preceding claim, further comprising an adhesive.

10. The hemostatic tape of any preceding claim wherein the tape is in a roll.

11. The hemostatic tape of any preceding claim further comprising at least one additional hemostatic agent.

12. The hemostatic tape of claim 11, wherein the at least one additional hemostatic agent is selected from the group consisting of fibrin, fibrinogen, collagen, albumin, clotting factors, albumin, elastn, thrombin, thrombinogen, dextran, alginates, cellulose, and combinations thereof.

13. The hemostatic tape of any preceding claim further comprising at least one barb.

## Patentansprüche

1. Hämostatischer Verbandstreifen, umfassend:
ein poröses Substrat (20, 120, 220, 320, 420, 520, 620) mit einer Faser- oder einer Schaumstoffstruktur;
eine erste Hydrogel-Vorstufe (30, 130, 230, 330, 430, 530, 630) und eine zweite Hydrogel-Vorstufe (40, 140, 240, 340, 440, 540, 540, 640), die auf das poröse Substrat aufgebracht sind; und
einen Abziehstreifen (60a, 360a), der auf wenigstens einer Seite des porösen Substrats angeordnet ist,
**dadurch gekennzeichnet, dass** die erste Hydrogel-Vorstufe auf einen ersten Abschnitt (22, 322, 422, 522, 622) des porösen Substrats aufgebracht ist und die zweite Hydrogel-Vorstufe auf einen zweiten Abschnitt (24, 324, 424, 524, 624) des porösen Substrats aufgebracht ist.

2. Hämostatischer Verbandstreifen nach Anspruch 1, wobei das poröse Substrat ein Schaumstoff ist.

3. Hämostatischer Verbandstreifen nach Anspruch 1, wobei das poröse Substrat aus einem Fasermaterial gefertigt ist.

4. Hämostatischer Verbandstreifen nach Anspruch 1, wobei das poröse Substrat aus oxidierter Cellulose gefertigt ist.

5. Hämostatischer Verbandstreifen nach einem vorangehenden Anspruch, wobei die erste Hydrogel-Vorstufe aus der Gruppe bestehend aus Partikeln, einem Schaum, einem Film und Kombinationen davon ausgewählt ist.

6. Hämostatischer Verbandstreifen nach einem vorangehenden Anspruch, wobei die zweite Hydrogel-Vorstufe aus der Gruppe bestehend aus Partikeln, einem Schaum, einem Film und Kombinationen davon ausgewählt ist.

7. Hämostatischer Verbandstreifen nach einem vorangehenden Anspruch, wobei der Abziehstreifen aus einem Material auf Silikonbasis gefertigt ist.

8. Hämostatischer Verbandstreifen nach einem vorangehenden Anspruch, der weiterhin einen Trägerstreifen (370a) umfasst.

9. Hämostatischer Verbandstreifen nach einem vorangehenden Anspruch, der weiterhin einen Klebstoff umfasst.

10. Hämostatischer Verbandstreifen nach einem vorangehenden Anspruch, wobei der Verbandstreifen in einer Rolle bereitgestellt ist.

11. Hämostatischer Verbandstreifen nach einem vorangehenden Anspruch, der weiterhin wenigstens ein zusätzliches Hämostatikum umfasst.

12. Hämostatischer Verbandstreifen nach Anspruch 11, wobei das wenigstens eine zusätzliche Hämostatikum aus der Gruppe bestehend aus Fibrin, Fibrinogen, Kollagen, Albumin, Gerinnungsfaktoren, Albumin, Elastin, Thrombin, Prothrombin, Dextran, Alginaten, Cellulose und Kombinationen derselben ausgewählt ist.

13. Hämostatischer Verbandstreifen nach einem vorangehenden Anspruch, der weiterhin wenigstens einen Widerhaken umfasst.

## Revendications

1. Bande hémostatique comprenant :
un substrat poreux (20, 120, 220, 320, 420, 520, 620) ayant une structure fibreuse ou alvéolaire ;
un premier précurseur d'hydrogel (30, 130, 230, 330, 430, 530, 630) et un second précurseur d'hydrogel (40, 140, 240, 340, 440, 540, 640) appliqués au substrat poreux ; et
une feuille antiadhésive (60a, 360a) disposée sur au moins une face du substrat poreux,
**caractérisée en ce que** le premier précurseur d'hydrogel est appliqué à une première partie (22, 322, 422, 522, 622) du substrat poreux et le second précurseur d'hydrogel est appliqué à une seconde partie (24, 324, 424, 524, 624) du substrat poreux.

2. Bande hémostatique selon la revendication 1, dans laquelle le substrat poreux est une mousse.

3. Bande hémostatique selon la revendication 1, dans laquelle le substrat poreux est constitué d'un matériau fibreux.

4. Bande hémostatique selon la revendication 1, dans laquelle le substrat poreux est constitué de cellulose oxydée.

5. Bande hémostatique selon l'une quelconque des revendications précédentes, dans laquelle le premier précurseur d'hydrogel est choisi dans le groupe constitué de particules, d'une mousse, d'un film et de leurs combinaisons.

6. Bande hémostatique selon l'une quelconque des revendications précédentes, dans laquelle le second précurseur d'hydrogel est choisi dans le groupe constitué de particules, d'une mousse, d'un film et de leurs combinaisons.

7. Bande hémostatique selon l'une quelconque des revendications précédentes, dans laquelle la feuille antiadhésive est constituée d'un matériau à base de silicone.

8. Bande hémostatique selon l'une quelconque des revendications précédentes comprenant en outre une feuille de support (370a).

9. Bande hémostatique selon l'une quelconque des revendications précédentes comprenant en outre un adhésif.

10. Bande hémostatique selon l'une quelconque des revendications précédentes, dans laquelle la bande se présente sous la forme d'un rouleau.

11. Bande hémostatique selon l'une quelconque des revendications précédentes comprenant en outre au moins un agent hémostatique supplémentaire.

12. Bande hémostatique selon la revendication 11, dans laquelle le au moins un agent hémostatique est choisi dans le groupe constitué de la fibrine, d'un fibrinogène, du collagène, de facteurs de coagulation, de l'albumine, de l'élastine, de la thrombine, du dextranne, des alginates, de la cellulose et de leurs combinaisons.

13. Bande hémostatique selon l'une quelconque des revendications précédentes comprenant en outre au moins un barbillon.
